# FASCICULE DE BREVET EUROPEEN

(11) **EP 2 288 313 B1**
(45) Date de publication et mention de la délivrance du brevet: **11.06.2014**
(21) Numéro de dépôt: 09735135.7
(22) Date de dépôt: 02.04.2009
(51) Int. Cl.: A61F 2/44, A61F 2/46

(54) **IMPLANT NUCLEIQUE**
NUCLEUS-PULPOSUS-IMPLANTAT
NUCLEUS PULPOSUS IMPLANT

(30) Priorité: 04.04.2008 FR 0801860
(43) Date de publication de la demande: 02.03.2011
(73) Titulaire: Clariance, 62000 Dainville (FR)
(72) Inventeur: TORNIER, Alain, F-38330 Saint-Ismier (FR); STEIB, Jean-Paul, F-67100 Strasbourg (FR); MAZEL, Christian, F-92350 Le Plessis Robinson (FR)
(74) Mandataire: Schmitt, John
(86) Numéro de dépôt international: PCT/FR2009/000389
(87) Numéro de publication internationale: WO 2009/130417

(56) Documents cités:
- WO-A-2004/082526
- WO-A-2006/047645
- WO-A-2007/022194
- WO-A-2008/005141
- WO-A1-02/11629
- WO-A1-2004/108022
- US-A1- 2007 150 059
- US-A1- 2007 168 041
- US-A1- 2007 233 146
- US-A1- 2007 255 282

## Description

La présente invention est relative à un implant nucléique à fil constituant entre deux vertèbres sus et sous jacentes d'un segment rachidien un dispositif d'étaiement intervertébral amortissant et assurant la mobilité de l'unité fonctionnelle formée par lesdites vertèbres.

### Art antérieur

La technique de remplissage par un fil fin est employée couramment pour le traitement des anévrismes artériels. Dans ce cas le comblement vise à obtenir la coagulation de la poche sanguine afin d'éviter sa rupture. Cette technique est utilisée en neuroradiologie interventionnelle depuis plus de 20 ans, sous le nom de technique des coils (spires de platine).

Une spirale de bande de matériel viscoélastique a déjà été introduite dans le disque par le professeur Husson. Cette bande est enfilée jusque dans l'espace nucléique au travers de l'annulus.

L'introduction d'un fil dans l'espace nucléique peut aisément se réaliser par abord percutané. Les voies d'abord percutanées trans-pédiculaires sont bien connues des chirurgiens et surtout des radiologues interventionnels notamment dans la mise en oeuvre des ballons de Kyphon dans les kyphoplasties.

Cette technique consiste à introduire un ballon par voie percutanée trans-pédiculaire dans une vertèbre fracturée et de le gonfler pour obtenir une reconstruction de la vertèbre et surtout un espace intra-vertébral dans lequel sera injecté un ciment après retrait du ballon.

L'abord trans-pédiculaire pour atteindre l'espace discal dans le cas de hernies a été décrit pour la première fois en 1978 par Patterson et Arbit. Cette technique a été ensuite améliorée avec l'utilisation d'un endoscope qui a permis d'obtenir la visualisation de l'espace interne du disque.

On connaît d'après la demande de brevet WO 2006/129027 un élément de remplissage destiné à remplir une cavité de corps vertébral pour la réalisation d'une prothèse de nucléus d'un disque intervertébral. L'élément de remplissage est préalablement chauffé à une température supérieure à celle du corps humain pour permettre son introduction sous la forme d'un filament souple venant former un enchevêtrement remplissant la cavité.

On connait d'après la demande de brevet WO 2008/005141 un implant vertébral constitué par exemple d'un ressort hélicoïdal introduit entre les vertèbres et dont les extrémités viennent s'engager dans les plateaux supérieur et inférieur des vertèbres sus et sous jacente. L'implant vertébral du fait de son profil hélicoïdal peut se déformer en compression et en friction.

La présente invention concerne un implant nucléique constituant entre deux vertèbres sus et sous jacentes Va, Vb d'un segment rachidien Sr un dispositif d'étaiement intervertébral amortissant et assurant la mobilité de l'unité fonctionnelle formée par lesdites vertèbres d'une colonne vertébrale.

L'implant nucléique suivant la présente invention est constitué d'au moins un élément de remplissage comportant au moins un fil continu agencé, à l'intérieur d'un espace nucléique Es obtenu après nucléotomie du disque intervertébral Di, suivant un profil en forme de couronne dont l'empilement des spires permet de délimiter un espace interne central, tandis que ledit espace interne central est rempli d'un produit qui peut être un gel ou un produit pâteux ou un produit à base de fibres ou un matériau viscoélastique injectable.

L'implant nucléique suivant la présente invention est constitué d'un élément de remplissage comportant d'une part un premier fil formant, à l'intérieur d'un espace nucléique Es obtenu après nucléotomie du disque intervertébral Di, une première couronne et d'autre part à l'intérieur de l'espace interne central délimité par la première couronne un second fil agencé pour réaliser une pelote.

L'implant nucléique suivant la présente invention est constitué d'un élément de remplissage comportant d'une part un premier fil formant, à l'intérieur d'un espace nucléique Es obtenu après nucléotomie du disque intervertébral Di, une première couronne et d'autre part à l'intérieur de l'espace interne central délimité par la première couronne un second fil agencé pour réaliser une autre couronne.

L'implant nucléique suivant la présente invention comporte un fil comprenant desprincipes actifs favorisant la formation de fibroses à l'intérieur de l'élément de remplissage.

L'implant nucléique suivant la présente invention comporte un fil radio opaque permettant de contrôler le remplissage et l'agencement en forme de couronne du fil à l'intérieur de l'espace nucléique Es.

L'implant nucléique suivant la présente invention comporte un fil qui est réalisé à partir de matériaux composites ou tressés et bio-résorbables.

L'implant nucléique suivant la présente invention comporte un fil agencé suivant un profil en forme de couronne qui présente un axe d'enroulement qui est sensiblement perpendiculaire aux plateaux des vertèbres Va, Vb du segment rachidien Sr.

L'implant nucléique suivant la présente invention comporte un fil comprenant une extrémité libre qui est affinée ou effilée pour éviter les blessures des tissus.

L'implant nucléique suivant la présente invention comporte un fil retenu à la vertèbre Va, Vb par l'intermédiaire d'un obturateur ou tout autre moyen pour éviter la migration dudit fil.

L'implant nucléique suivant la présente invention comprend au moins deux couronnes de fil disposées l'une à côte de l'autre dans le même espace nucléique Es.

Le dispositif d'introduction pour l'implant nucléique suivant la présente invention comporte une canule filetée ou un tube guide à profil courbé, une première ou seconde gaine flexible guide fil qui est introduite à l'intérieur de ladite canule filetée ou dudit tube guide à profil courbé, de manière que l'une des extrémités de la dite gaine flexible débouche à l'intérieur de l'espace nucléique Es, tandis que l'autre extrémité est raccordée à des moyens d'avancement ou instrument permettant l'introduction du fil à l'intérieur dudit espace nucléique Es.

Le dispositif d'introduction pour l'implant nucléique suivant la présente invention comporte une canule filetée pourvue à l'une de ses extrémités d'un filetage assurant son ancrage osseux dans la vertèbre Vb correspondante, d'un canal intérieur se terminant par une sortie latérale destinée à atteindre le plateau supérieur Ps de la vertèbre sous jacente Vb ou inférieure Pi de la vertèbre sus jacente Va et d'une tête comprenant sur sa périphérie un index de repérage permettant au chirurgien de visualiser la position de la sortie latérale du canal intérieur à l'intérieur de la vertèbre sus jacente ou sous jacente Vb et un prolongement assurant la connexion de ladite canule.

Les dessins annexés, donnés à titre d'exemple, permettront de mieux comprendre l'invention, les caractéristiques qu'elle présente et les avantages qu'elle est susceptible de procurer :
Figure 1 est une vue en perspective éclatée illustrant l'implant nucléique suivant la présente invention dont l'élément de remplissage est constitué d'un fil disposé en couronne.
Figure 2 est une vue en coupe illustrant l'implant nucléique suivant la présente invention dont l'élément de remplissage est constitué de deux fils disposés en couronne.
Figures 3 à 17 sont des vues montrant un exemple de réalisation des différentes étapes permettant l'introduction de l'implant nucléique par une voie d'abord transpédiculaire au niveau de la vertèbre sus jacente Va d'un segment rachidien Sr suivant le présente invention.
Figure 18 est une vue représentant l'introduction d'un produit réalisé sous la forme d'un gel ou d'un produit pâteux ou d'un produit à base de fibres ou d'un matériau viscoélastique injectable à l'intérieur de l'espace interne central formé par la couronne du premier fil de l'implant nucléique par voie d'abord transpédiculaire au niveau de la vertèbre sus jacente Va suivant le présente invention.
Figures 19 à 33 sont des vues représentant un autre exemple de réalisation des différentes étapes permettant l'introduction de l'implant nucléique par voie d'abord transpédiculaire au niveau de la vertèbre sous jacente Vb d'un segment rachidien Sr suivant le présente invention.
Figures 34 à 36 sont des vues illustrant l'introduction d'un second fil à l'intérieur de l'espace interne central formé par la couronne du premier fil de l'implant nucléique par voie d'abord transpédiculaire suivant le présente invention.

### Implant Nucléique

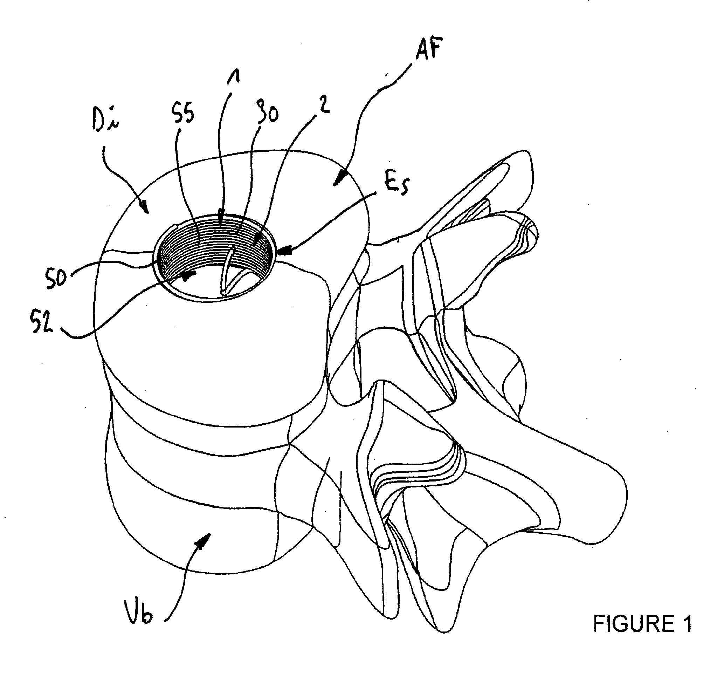

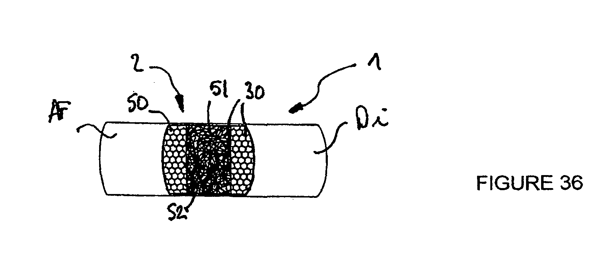

On a illustré en figures 1, 2, 18 et 34 à 36 différentes réalisations, d'agencement et de constitution d'un implant nucléique 1 suivant la présente invention. A vérifier

L'implant nucléique 1 est constitué d'au moins un élément de remplissage 2 comportant au moins un fil 50 continu qui est agencé en forme de couronne et dont l'empilement des spires 55 permet de délimiter un espace interne central 52, lorsque lesdites spires viennent s'enrouler à l'intérieur d'un espace nucléique Es préalablement aménagé dans le disque intervertébral Di de deux vertèbres sus et sous jacentes Va, Vb d'un segment rachidien Sr.

L'implant nucléique 1 est constitué d'un fil 50 dont l'enroulement quasi jointif des spires 55 en forme de couronne délimite un espace interne central 52 et un certain nombre d'interstices 30 entre chaque spire.

La couronne de spires 55 est formée par plusieurs couches jointives de fil 50 dont l'axe d'enroulement est sensiblement perpendiculaire aux plateaux des vertèbres Va, Vb du segment rachidien.

A l'intérieur de l'espace interne central 52 délimité par l'enroulement du fil 50 peut être disposé un second fil 51 agencé soit suivant une seconde couronne, soit suivant une pelote, soit suivant les deux et délimitant d'autres interstices 30 permettant de réaliser l'élément de remplissage 2 (Figures 1, 2, 36).

Le ou les fils 50, 51 sont prévus biocompatibles, de faible diamètre ou de diamètres différents en fonction de la constitution de l'implant nucléique 1. En ce qui concerne la réalisation d'un implant nucléique 1 au moyen d'un seul fil 50 agencé en couronne, ce dernier présente un diamètre externe qui est compris, par exemple, entre 0,4 et 0,8 millimètres.

Le ou les fils 50, 51 peuvent être inertes ou comporter des principes actifs ayant pour but de favoriser le développement de tissu fibrotique à l'intérieur de l'espace nucléique Es préalablement aménagé dans le disque intervertébral Di de deux vertèbres sus et sous jacentes Va, Vb d'un segment rachidien Sr

Le ou les fils 50, 51 sont réalisés à partir de matériaux composites tressés ou non bio-résorbables contenant ou non des principes actifs destinés à favoriser la constitution d'une fibrose réalisant ainsi un « Néonucléus ».

Le ou les fils 50, 51 sont radio-opaques permettant de contrôler leur introduction dans l'espace nucléique Es en per-opératoire et le suivi de l'implant nucléique 1 en post-opératoire.

Le ou les fils 50, 51 comportent une extrémité libre, c'est à dire celle pénétrant à l'intérieur de l'espace nucléique Es, qui est affinée ou effilée et courbée pour éviter les blessures des tissus du fait de sa faible résistance au contact notamment lors de son introduction dans l'espace nucléique Es.

L'implant nucléique 1 comporte selon son agencement en forme de couronne un nombre important d'interstices 30, de dimensions différentes, qui sont obtenus par l'empilement des spires 55 et favorisant la formation d'une fibrose à l'intérieur de l'espace nucléique Es.

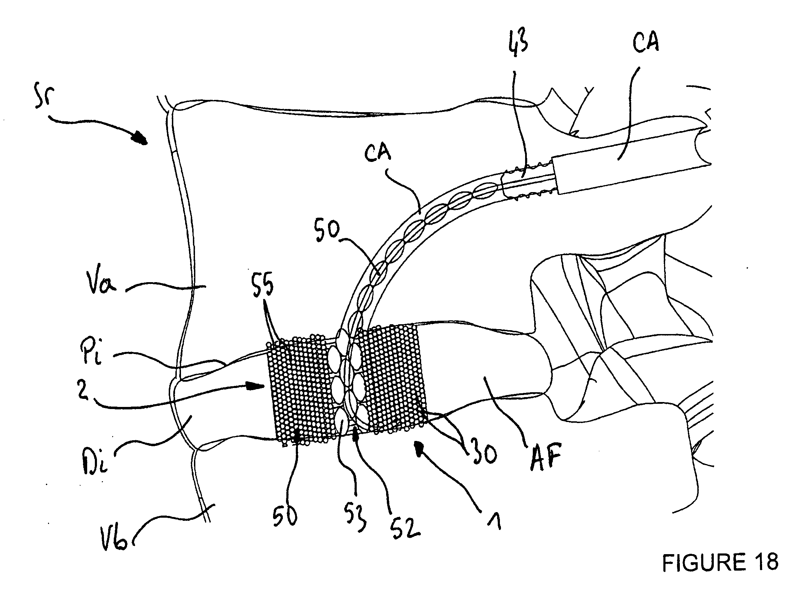

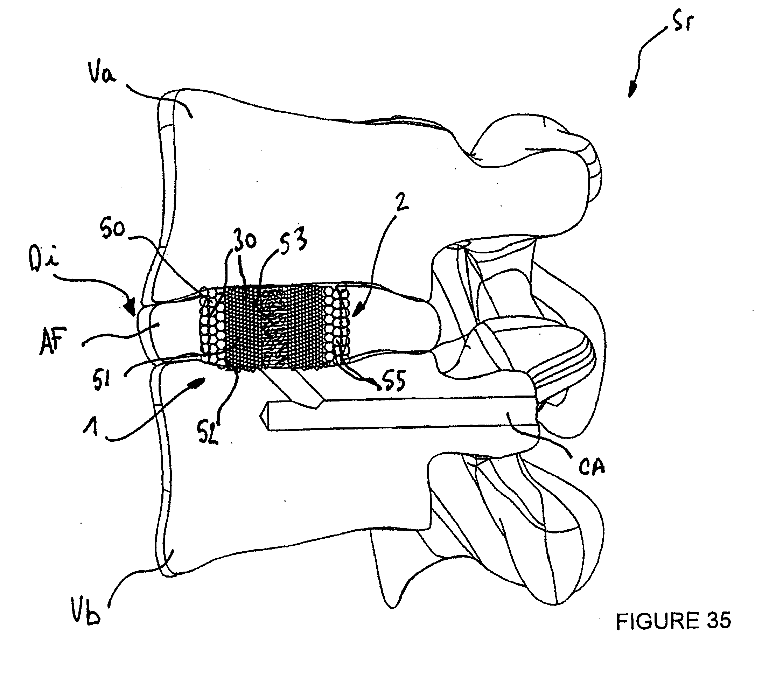

L'implant nucléique 1 peut comporter, à l'intérieur de l'espace interne central 52 réalisé à partir du fil 50 ou de l'agencement des fils 50, 51, un produit 53 qui peut être un gel ou un produit pâteux ou un produit à base de fibres ou un matériau viscoélastique injectable sous forme visqueuse ou liquide afin de constituer un pseudo-nucléus (Figures 18, 35).

L'élément de remplissage 2 ainsi obtenu forme un implant nucléique 1 de type « néo-nucléus » ayant de bonnes caractéristiques mécaniques, de cohésion et d'amortissement lorsqu'il est constitué par exemple d'une première couronne de fil 50 formant un « pseudo-annulus » associée soit à une deuxième couronne ou pelote de fil 51, soit à un produit 53 qui peut être gel ou un produit pâteux ou un produit à base de fibres ou un matériau viscoélastique injectable formant un « pseudo-nucléus ».

Il a été observé lorsqu'une compression est exercée sur l'élément de remplissage 2 que ce dernier entraîne des efforts de contact sur les fils 50, 51, les verrouillant entre eux. Ce verrouillage donne à l'implant nucléique 1 une consistance permettant un bon comportement mécanique aux efforts de compression et une tenue de la structure. Plus cette structure est dense, plus le comportement de l'élément de remplissage 2 est rigide.

Cet élément de remplissage 2 permet d'obtenir une bonne résistance à la compression nécessaire pour un implant nucléique 1 tout en limitant les efforts latéraux. La limitation des efforts latéraux notamment sur « l'annulus fibrosus » (AF) est un point clef pour un tel implant afin d'éviter les phénomènes de compression nerveuse pouvant survenir par le bombement externe de « l'annulus fibrosus » (AF) du disque intervertébral Di ou par une hernie discale.

Ainsi l'implant nucléique 1 suivant la présente invention forme une masse conséquente remplissant l'espace discal obtenu après nucléotomie.

Le ou les fil 50, 51 présentent une rigidité suffisante pour pouvoir former une couronne constituée par l'empilement de spires 55 quasi jointives qui se lovent, par effet de flambement, à l'intérieur de l'espace nucléique Es.

L'implant nucléique 1 suivant la présente invention ainsi constitué forme une masse conséquente remplissant l'espace discal Es obtenu après nucléotomie.

L'implant nucléique 1 suivant la présente invention peut comporter au moins deux couronnes de fil 50 disposées l'une à côte de l'autre dans le même espace nucléique Es. Dans cas particulier, on réalise deux abords transpédiculaires au niveau de chaque pédicule de la même vertèbre.

### Nucléotomie du disque intervertébral

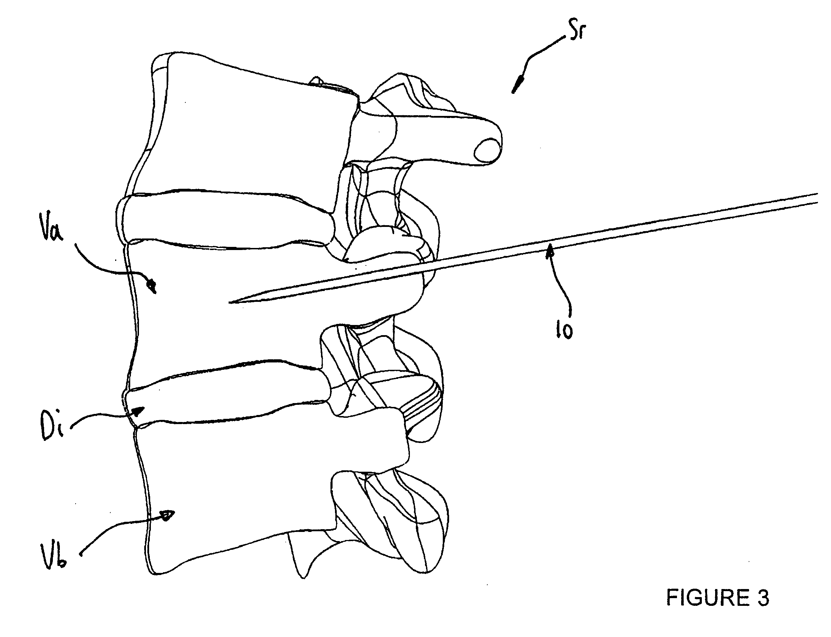

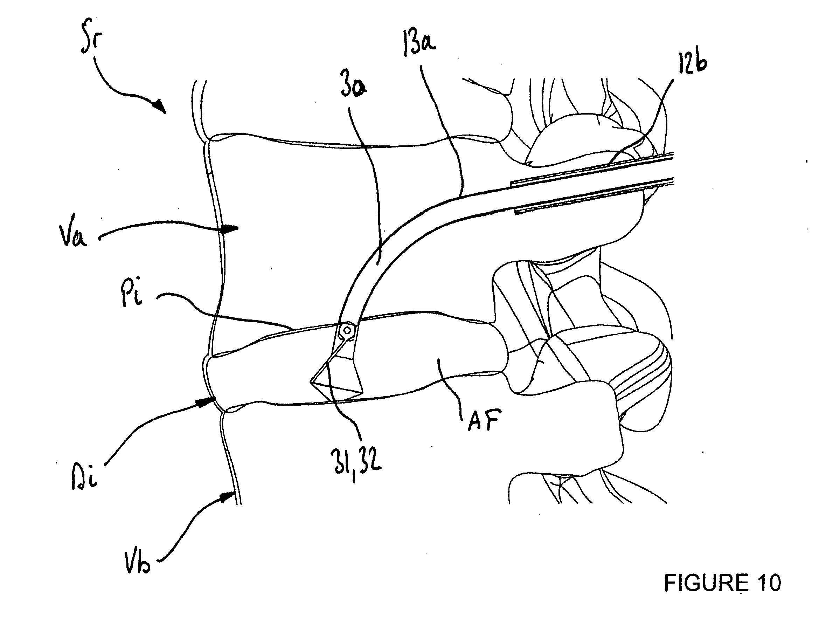

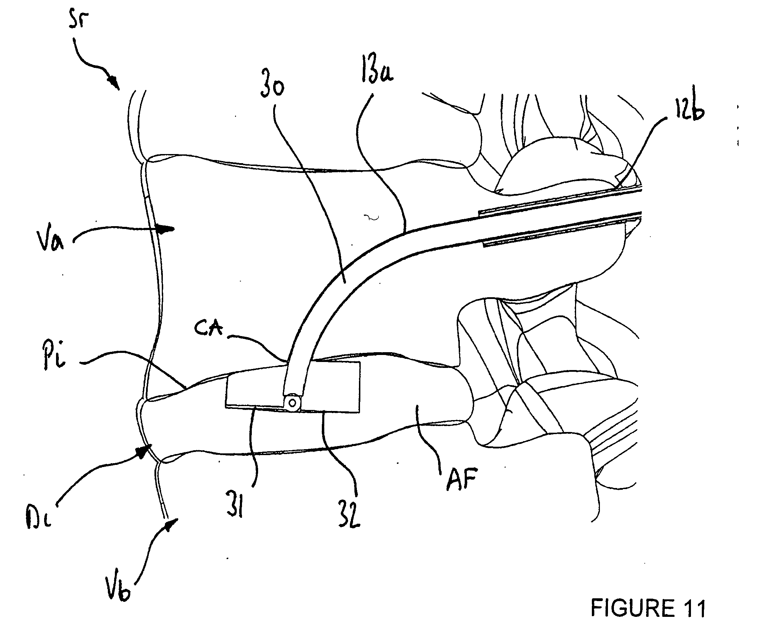

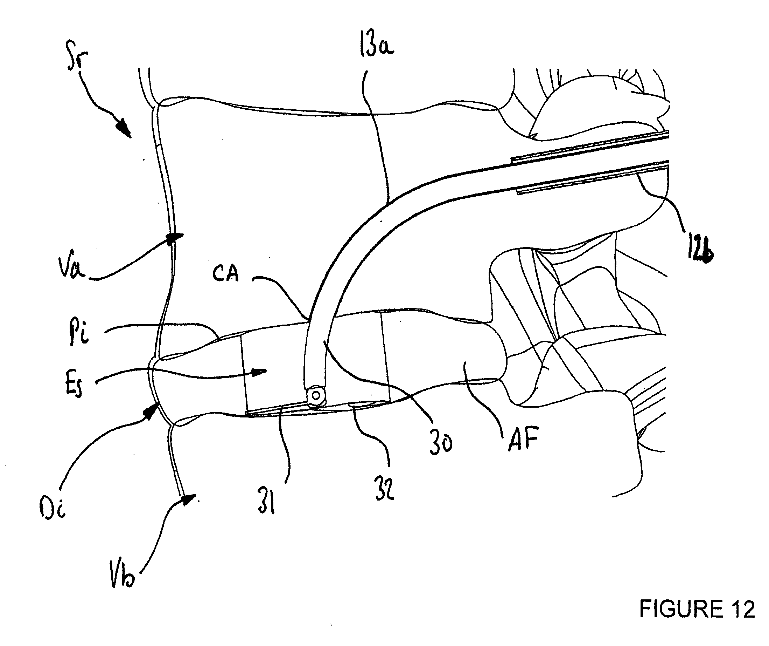

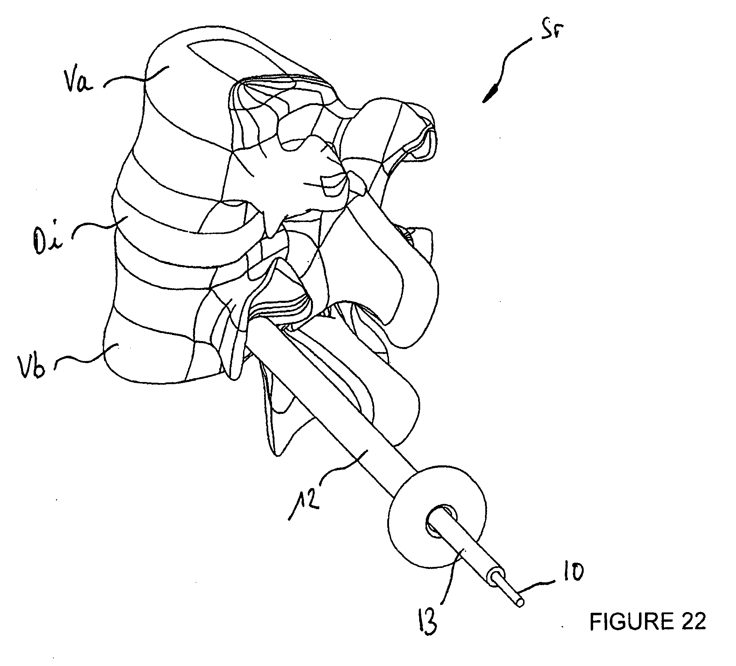

On a montré en figures 3 à 12 et 19 à 29 deux vertèbres sus et sous jacentes Va, Vb d'un segment rachidien Sr d'une colonne vertébrale dont le disque intervertébral Di va subir une nucléotomie par abord trans-pédiculaire.

Cette nucléotomie du disque intervertébral Di va permettre l'introduction d'un implant nucléique 1 dont la structure en forme de couronne comporte un espace interne central 52 délimité par l'enroulement des spires 55.

A cet effet, le disque intervertébral Di comporte une structure interne désignée « nucleus pulposus » (NP) qui est entourée par un anneau fibreux dénommé « annulus fibrosus » (AF).

La composition biochimique du « nucleus pulposus » (NP) est en grande majorité constituée d'un hydrogel dans lequel les molécules dominantes sont constituées de glycosaminoglycannes, au sein desquelles sont suspendues des cellules particulières au « nucleus pulposus » (NP). L'environnement biologique in situ est plutôt acide et anaérobie, ce qui est défavorable à la croissance cellulaire.

Les glycosaminoglycannes sont des polymères biologiques constitués de longues chaînes glycosidiques (le monomère constitutif est un membre de la famille des sucres), comportant des dérivés animés ou peptidiques d'où leur autre dénomination de protéoglycanne. Ces polymères sont en général très hydrophiles.

Les glycannes constituent la matrice dans laquelle les cellules sont maintenues. La matrice est en contact très étroit avec la zone ostéochondrale des plateaux vertébraux inférieurs et supérieurs (craniaux et caudaux) des vertèbres sus et sous jacentes Va, Vb. Cette zone est la seule permettant les rares échanges nutritifs avec le « nucleus pulposus » (NP).

Les cellules présentes dans le « nucleus pulposus » (NP), quoiqu'en très faible quantité, sont issues des cellules embryonnaires à savoir des cellules de type « chondrocytaires » et des cellules plus grandes, vacuolées issues des lignées notochordales.

Ces dernières diminuent rapidement avec l'âge, de même leur métabolisme est affecté par les conditions acides qui apparaissent avec le stress mécanique et diminuent leur potentiel à régénérer la matrice.

Ainsi, le stress mécanique dû aux millions de cycles de contraintes sur le « nucleus pulposus » (NP) génère des conditions acidiques (lactate) qui empêcheraient les cellules de renouveler le stock de matrice.

La pénétration par un plateau des vertèbres sus ou sous jacente Va, Vb du segment rachidien Sr dans le « nucleus pulposus » (NP) du disque intervertébral Di permet de réaliser la nucléotomie de ce dernier tout en permettant la diffusion de cellules sanguines dans l'espace nucléique Es favorisant les conditions d'une formation d'une fibrose.

### Introduction de l'implant nucléique 1 à un fil 50 par une voie d'abord transpédiculaire au niveau de la vertèbre sus jacente Va d'un segment rachidien Sr (figures 3 à 17)

La voie transpédiculaire présente les avantages suivants par rapport à la voie classique annulaire :
❖ Evite la dégradation de l'annulus par les instruments de nucléotomie,
❖ Ménage un abord quasi central et vertical par rapport au plateau de la vertèbre, et dans l'espace nucléique Es, ce qui est essentiel pour permettre l'introduction d'un fil 50, 51 pour constituer un étai intervertébral en forme de couronne d'axe vertical,
❖ Donne la possibilité de fermer l'espace nucléique Es par la fermeture du canal osseux transpédiculaire Ca par l'intermédiaire d'un obturateur ou tout autre moyen.

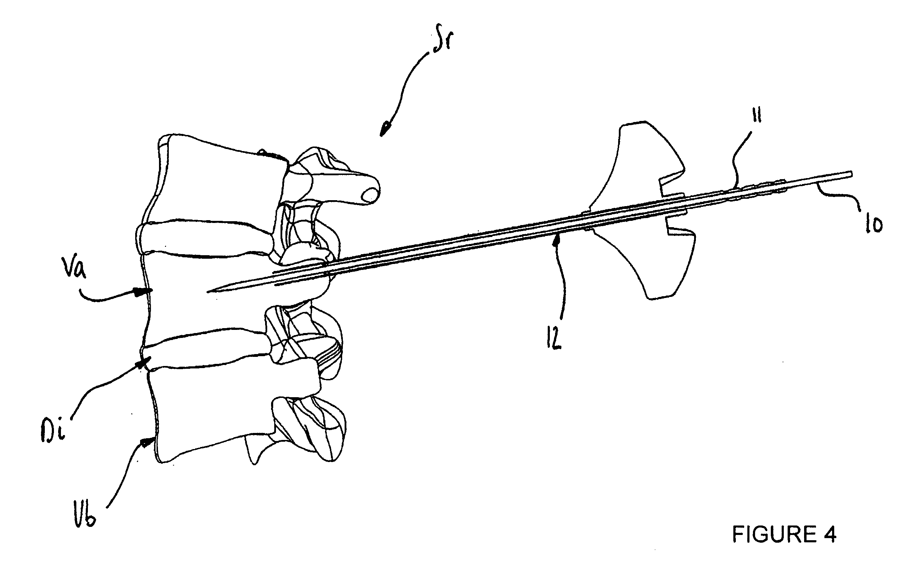

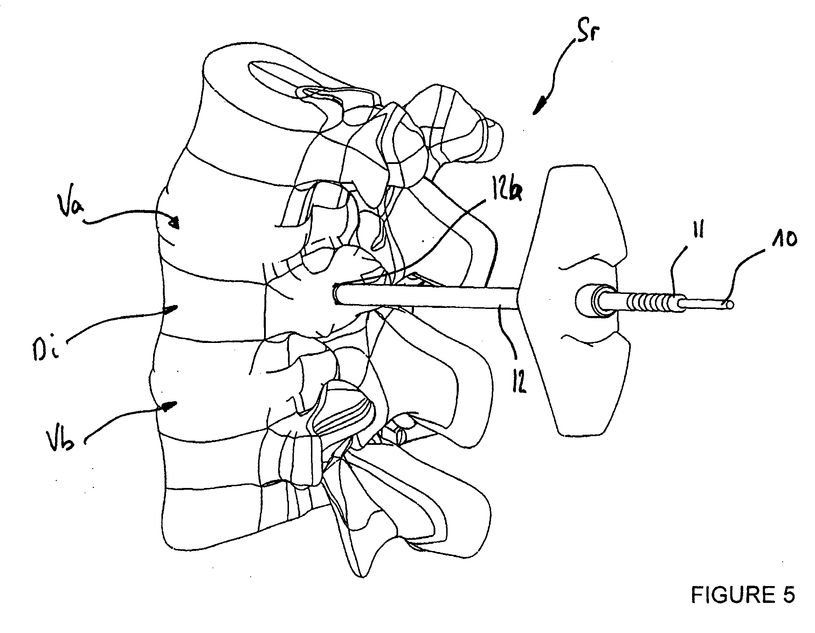

Ainsi, la visée pédiculaire, la nucléotomie du disque intervertébral Di et l'introduction de l'implant nucléique 1 sont conduites de la manière suivante par le chirurgien :
- Le chirurgien positionne et introduit une broche guide 10 sous contrôle radiologique dans le pédicule de la vertèbre sus jacente Va du segment rachidien Sr (Figure 3). L'introduction de la broche guide 10 est réalisé à partir d'un point d'entrée prévu au niveau d'un des deux pédicules de la vertèbre sus jacente Va et en visant le point du corps vertébral coupant la verticale passant par le centre du nucléus (NP).
- Le chirurgien enfile une bougie 11 sur la broche guide 10 pour atteindre le pédicule (Figure 4).
- Le chirurgien positionne sur la bougie 11 un tube guide ou canule droite 12 qui est pourvu à son extrémité d'une portion filetée 12b permettant un solide ancrage dans le corps de la vertèbre sus jacente Va (Figures 4 et 5). Ainsi la broche guide 10 définit le trajet de la canule droite 12 qui est introduite par vissage jusqu'au delà du mur postérieur du corps vertébral afin de matérialiser un canal osseux de guidage transpédiculaire Ca et sécuriser contre toute agression potentielle les éléments neurologiques. L'axe de la canule droite 12 passe à la verticale du centre du nucléus (NP) se situant en-dessous. L'axe de la canule droite 12 dans le plan sagittal, la position de son extrémité dans la vertèbre et les dimensions de la vertèbre Va elle même déterminent le profil d'un tube guide courbe ou d'une canule courbe 13a adapté(e) au cas.

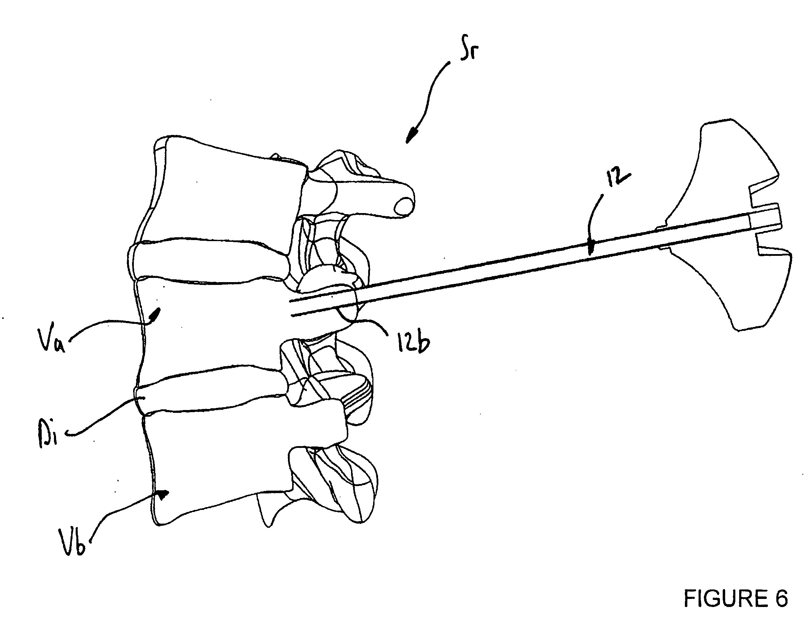

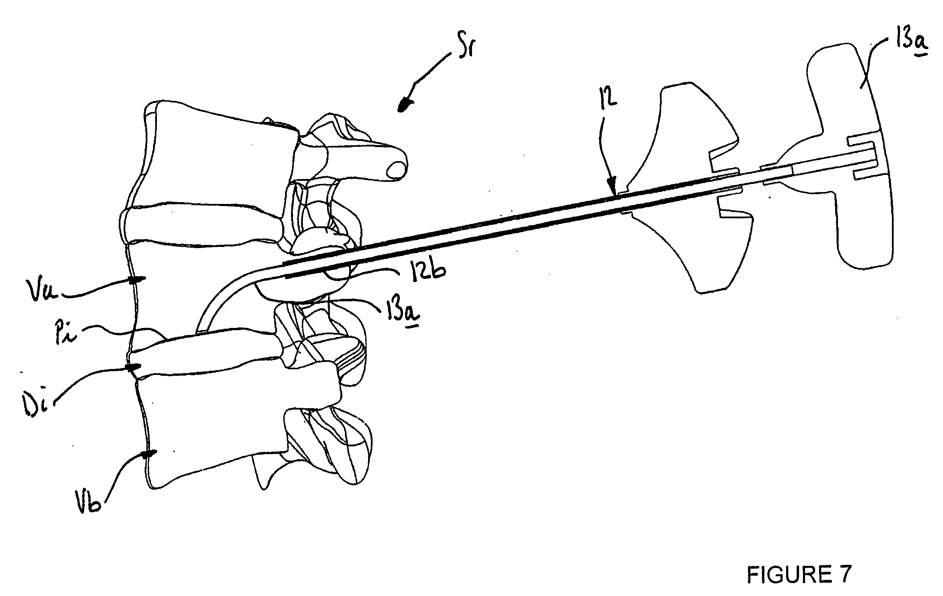

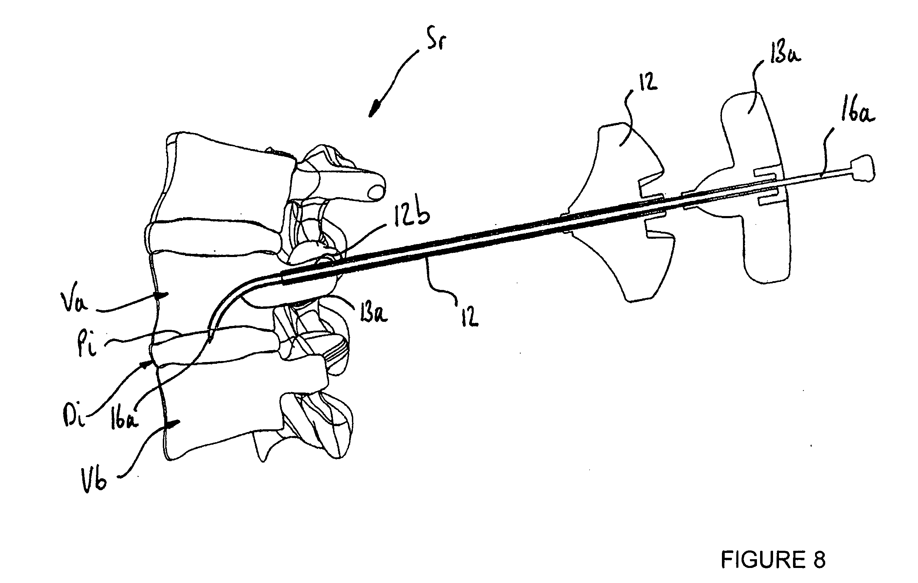

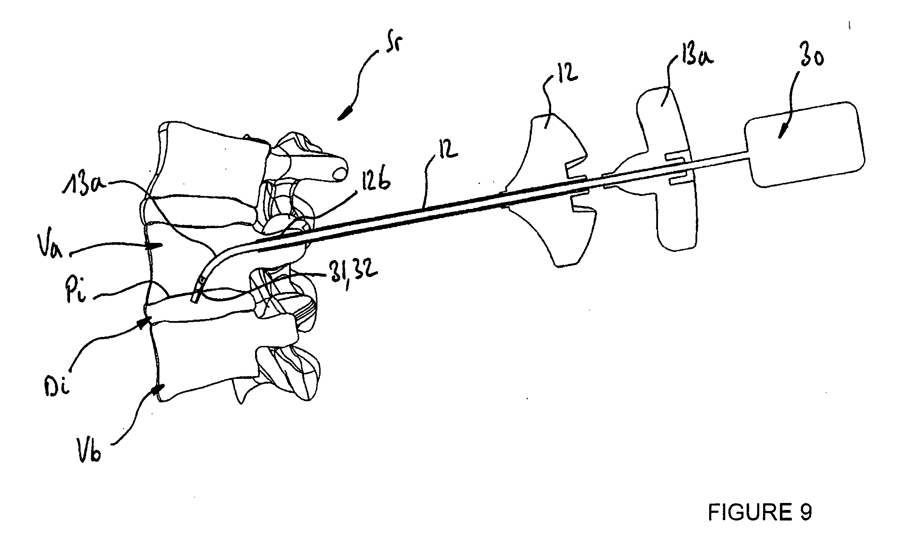

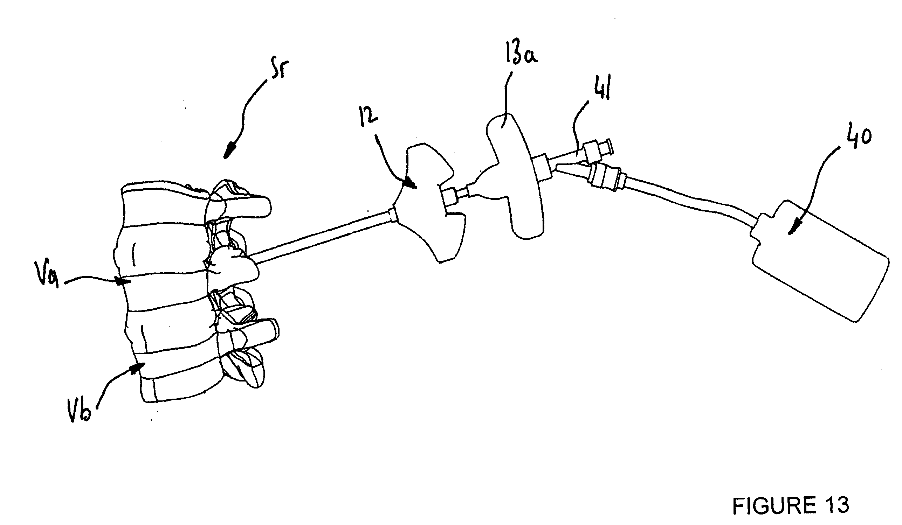

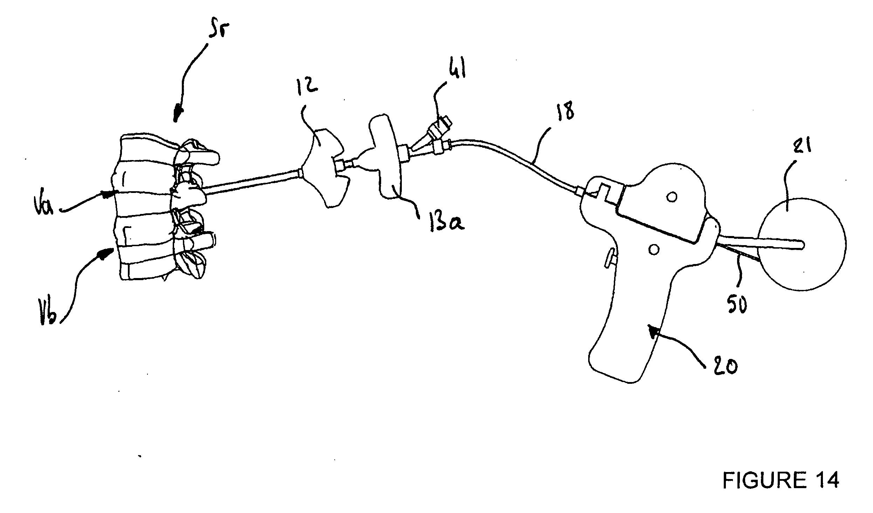

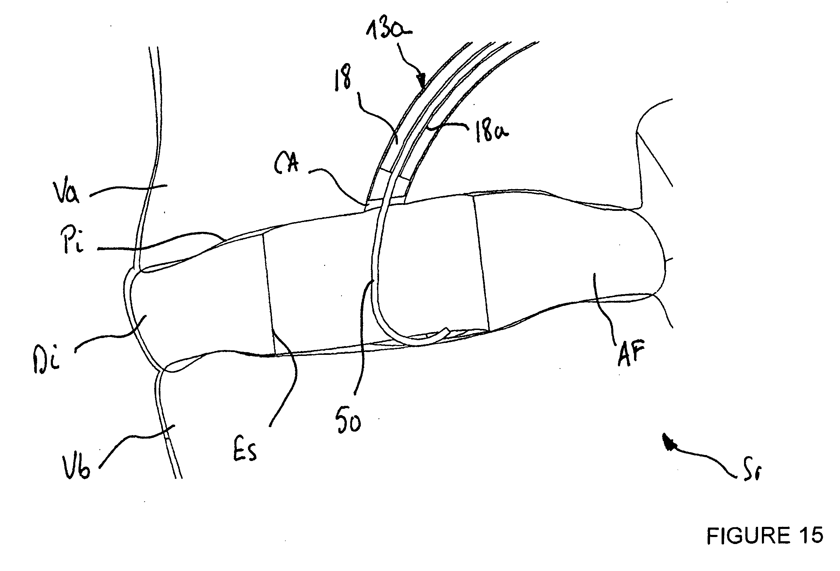

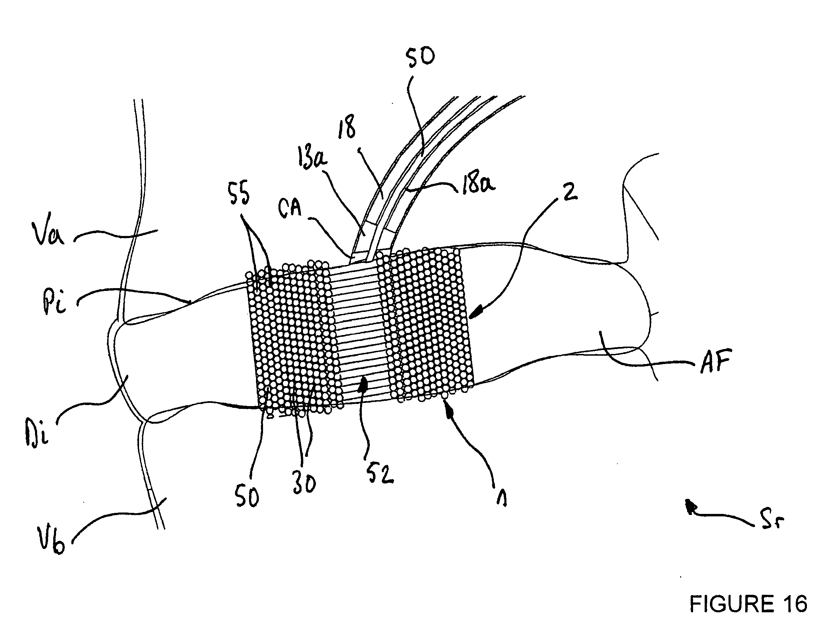

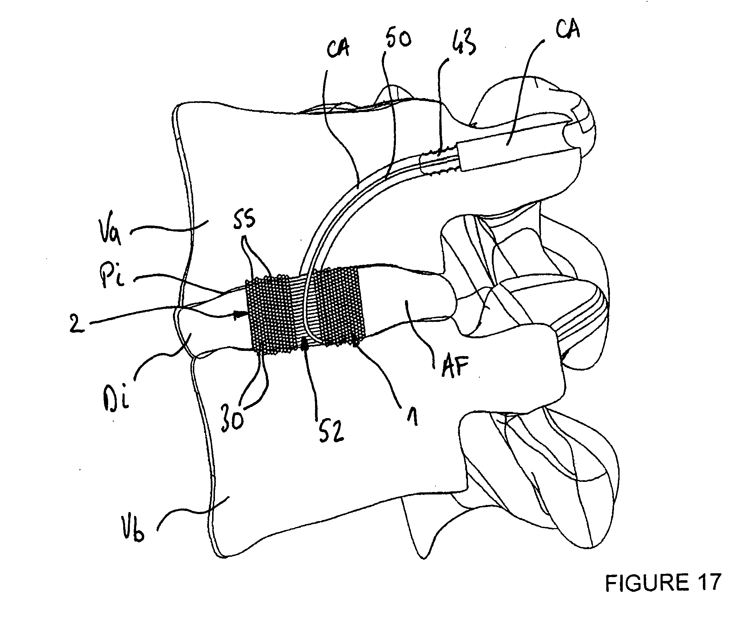

- Le chirurgien retire la broche 10 et la bougie 11 et introduit dans le tube guide ou canule droite 12 un second tube guide courbe ou canule courbe 13a permettant de ménager un canal osseux de guidage transpédiculaire Ca jusqu'au niveau du plateau inférieur Pi de la vertèbre sus jacente Va (figures 6, 7). La canule courbe 13a est réalisée en matériau super élastique et poussée progressivement au travers de la canule droite 12 pour sortir de cette dernière en se courbant jusqu'à atteindre le plateau inférieur Pi de la vertèbre Va. Le guidage obtenu par la canule droite 12 et le choix du profil de la canule courbe 13a permettent d'atteindre aisément le point sur le plateau Pi choisi lors de la planification pré opératoire.
- Le chirurgien introduit dans le tube guide courbe ou canule courbe 13a une fraise flexible ou impacteur flexible 16a permettant le perçage du plateau inférieur Pi de la vertèbre sus jacente Va (figure 8).
- Après le retrait de la fraise flexible 16a le chirurgien introduit sous contrôle radiologique dans le tube guide courbe ou canule courbe 13a un dispositif de coupe flexible ou une tige flexible de coupe 30 comportant par exemple à son extrémité des outils de coupe ou des petites lames de coupe 31, 32 rétractables qui sont actionné(e)s en rotation pour réduire les tissus de l'espace nucléique Es en débris évacuables par le canal osseux de guidage transpédiculaire Ca (figures 9 à 12).
- Le chirurgien procède sous contrôle radiologique au nettoyage de l'espace nucléique Es au moyen d'une canule flexible 42 introduite au travers du raccord étanche 41 dans la canule courbe 13a jusqu'à l'intérieur dudit espace nucléique Es et reliée à un système ou pompe 40 permettant l'injection et l'aspiration de liquide physiologique. L'aspiration du liquide par le système ou pompe 40 permet l'entraînement des débris hors de l'espace nucléique Es (figure 13).
- Dès que l'espace nucléique Es est réalisé, vidé et nettoyé le chirurgien peut procéder à l'introduction de l'implant nucléique 1 constitué de l'élément de remplissage 2 formé du fil 50 à l'intérieur dudit espace.
- Le chirurgien introduit au travers du raccord 41 et dans le tube guide courbe ou canule courbe 13a une gaine flexible guide fil 18 jusqu'à atteindre le plateau inférieur Pi de la vertèbre sus jacente Va pour déboucher à l'intérieur de l'espace nucléique Es. La gaine flexible 18 possède un alésage interne 18a qui correspond sensiblement au diamètre du fil 50 à introduire dans l'espace nucléique Es (Figures 14, 15).
- Le chirurgien connecte la gaine flexible 18 au moyen d'un système d'introduction de type « avance fil dans les postes de soudure » comportant par exemple un pistolet 20 pourvu d'un chargeur interne ou externe 21 dans lequel est enroulée une longueur suffisante de fil 50 (Figures 14).
- Le chirurgien agit sur les moyens d'entraînement du pistolet 20 provoquant une poussée du fil 50 à l'intérieur de l'alésage interne 18a de la gaine flexible 18 et ce jusqu'à l'intérieur de l'espace nucléique Es ménagé dans le disque intervertébral Di. Une certaine longueur de fil 50 est introduite dans l'espace nucléique Es avec une vitesse contrôlée. L'introduction en continu du fil 50 et son placement en forme de couronne dans l'espace nucléique Es sont suivie sous contrôle radiologique car le fil 50 est radio opaque. (Figures 15, 16). Le fil 50 ainsi poussé, s'enroule et tapisse les parois internes de « l'annulus fibrosus » (AF) en se lovant. En effet, le fil 50 possédant une certaine rigidité se courbe par effet de flambement et se place en couronne dans l'espace nucléique 1 en formant des rangées de spires jointives 55 sur toute la hauteur dudit espace. Le fil 50 en se lovant forme à l'intérieur de l'espace nucléique Es une couronne d'axe sensiblement vertical par rapport aux plateaux des vertèbres Va, Vb. La couronne de spires 55 délimite un espace central interne 52 pouvant être rempli par des produits complémentaires introduits en per-opératoire tel qu'un autre fil 51 ou un produit 53 qui peut être gel, un produit pâteux, un produit à base de fibres, un matériau viscoélastique injectable ou par la formation d'une fibrose en post-opératoire favorisée par la vascularisation due à l'abord transpédiculaire.
- Le chirurgien retire la gaine flexible 18, le système d'introduction 20, le tube guide courbe ou canule courbe 13a et le tube guide ou canule droite 12 du canal osseux Ca.
- Le chirurgien fixe l'extrémité du fil 50 constituant la couronne à la vertèbre Va à l'intérieur du canal osseux Ca par l'intermédiaire de moyens de fixation ou d'un obturateur 43 qui permet(tent) également d'obturer ledit canal osseux. Le fil 50 solidarise ainsi l'implant nucléique 1 à la vertèbre Va pour prévenir les problèmes potentiels de migration (Figure 17).

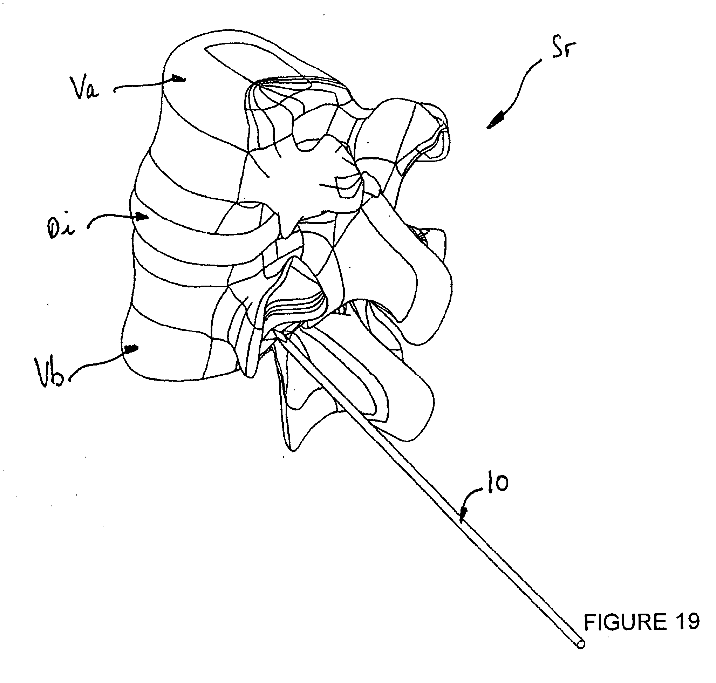

### Introduction de l'implant nucléique 1 à un fil 50 par une voie d'abord transpédiculaire au niveau de la vertèbre sous jacente Vb d'un segment rachidien Sr (figures 19 à 33)

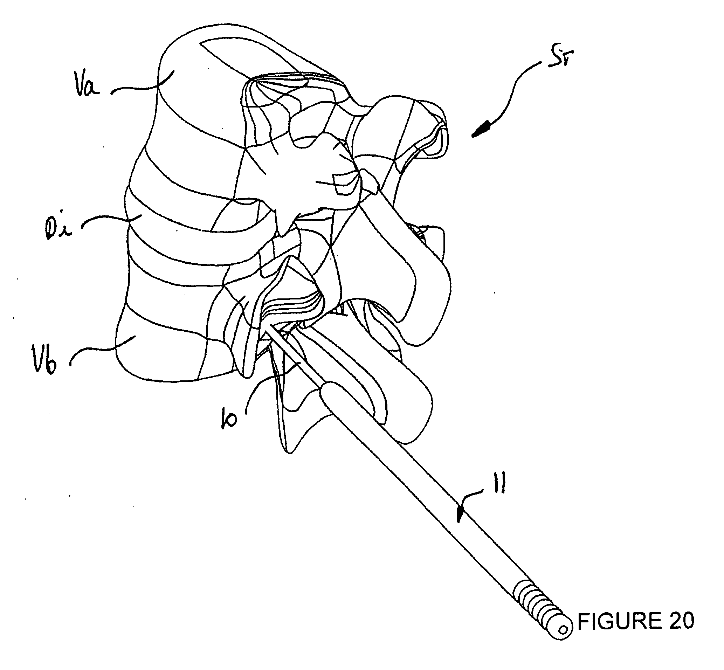

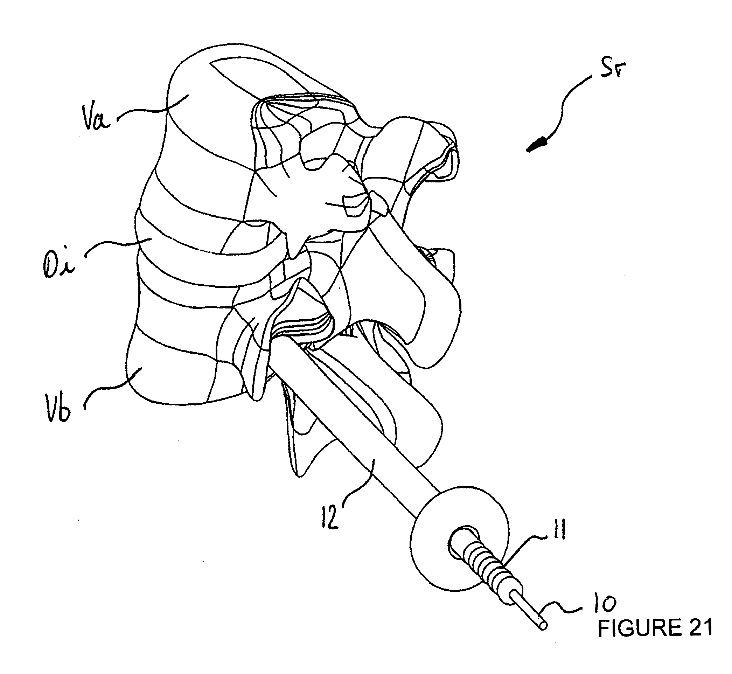

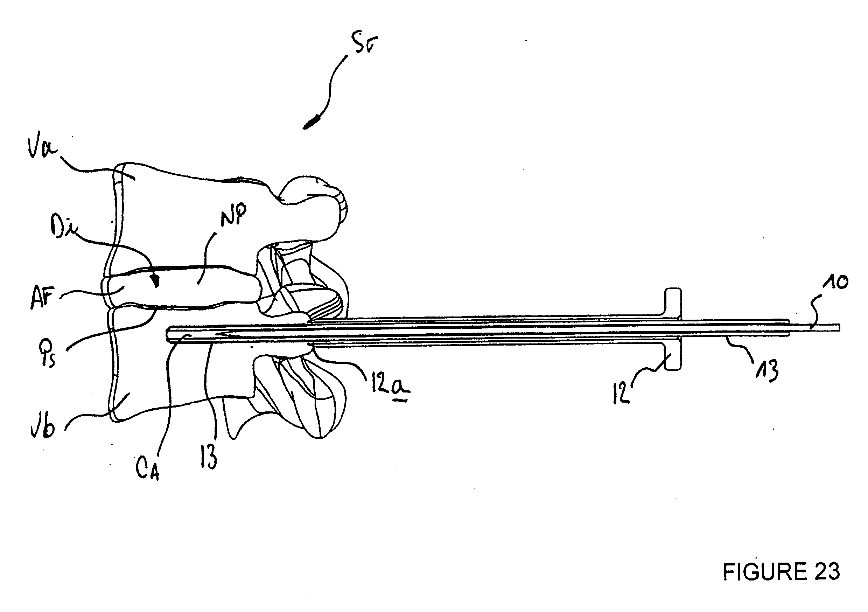

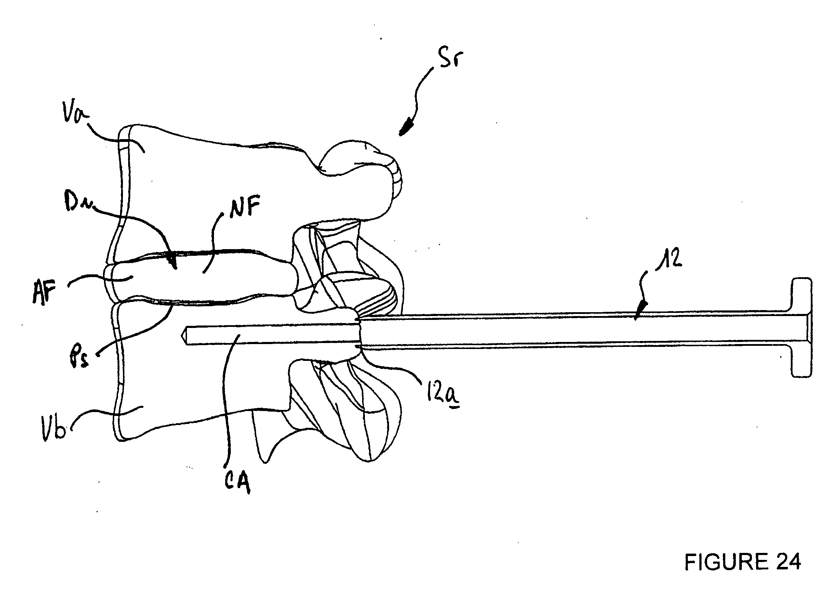

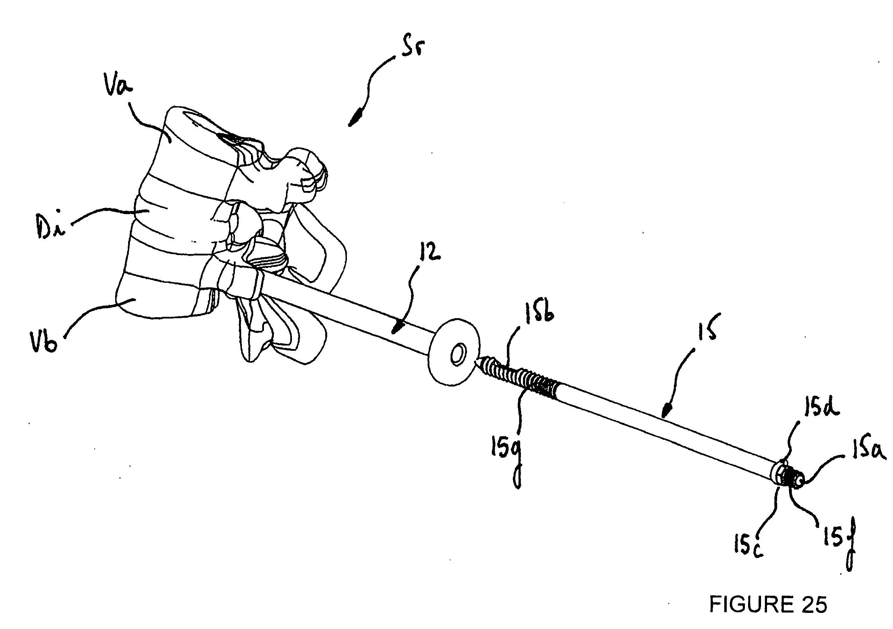

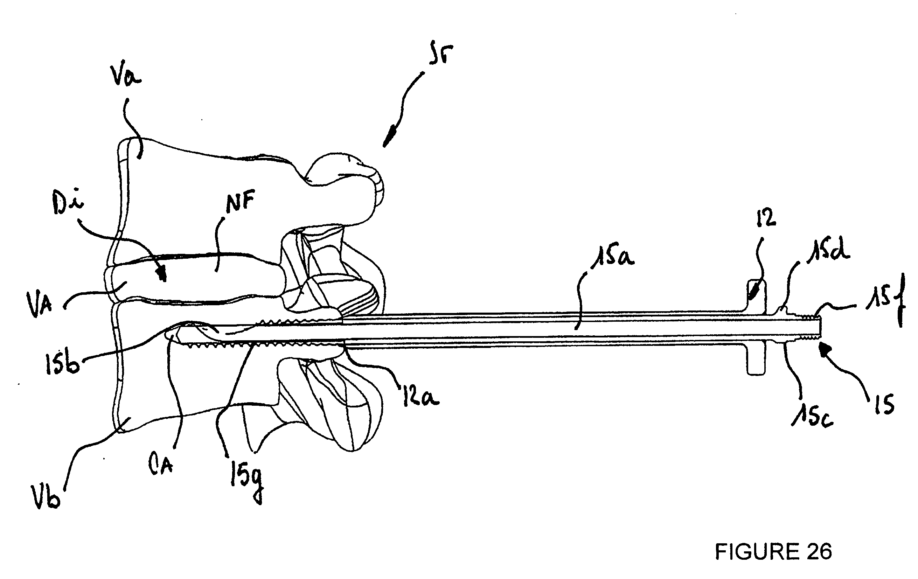

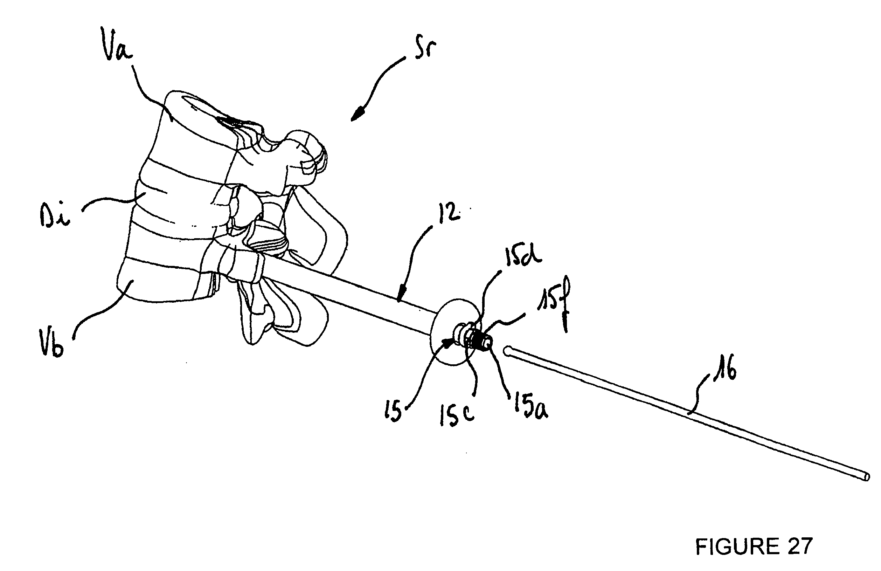

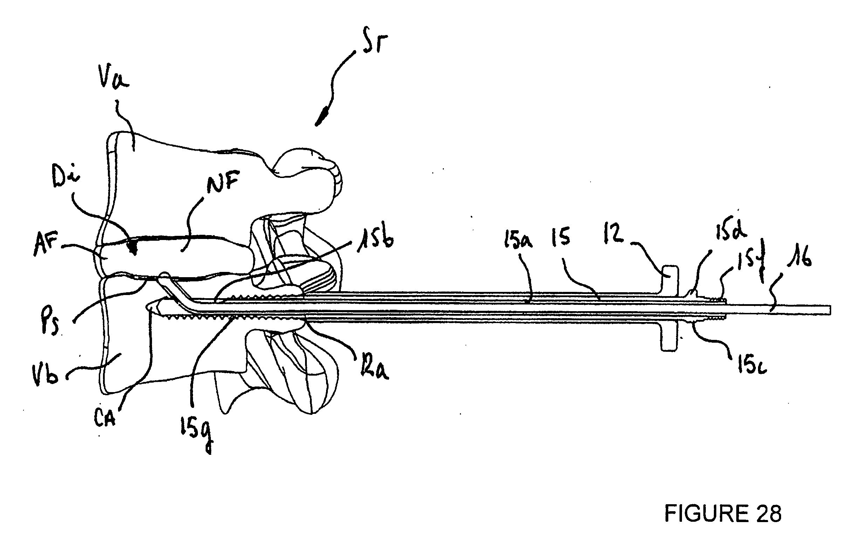

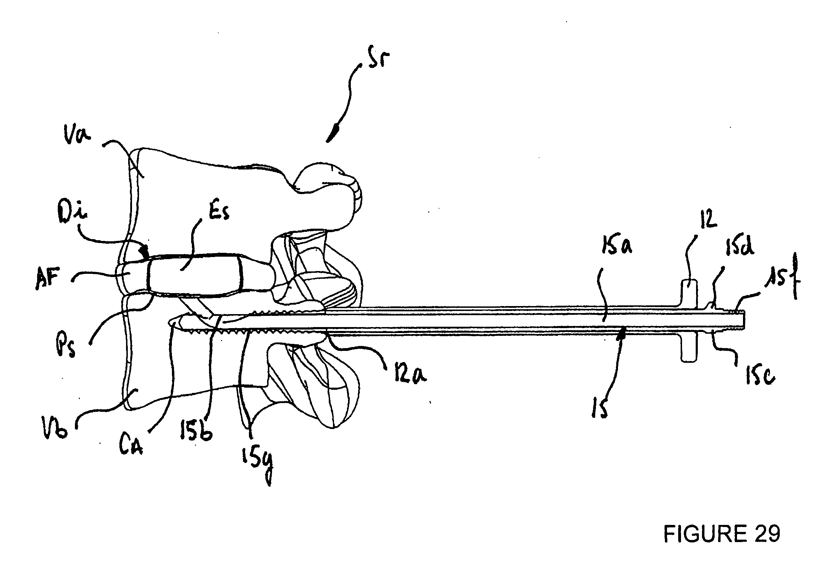

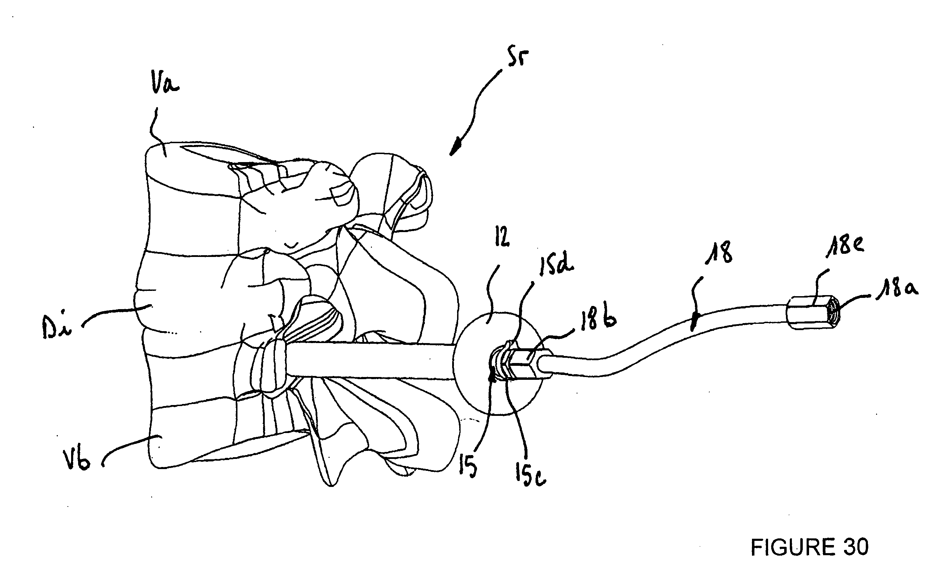

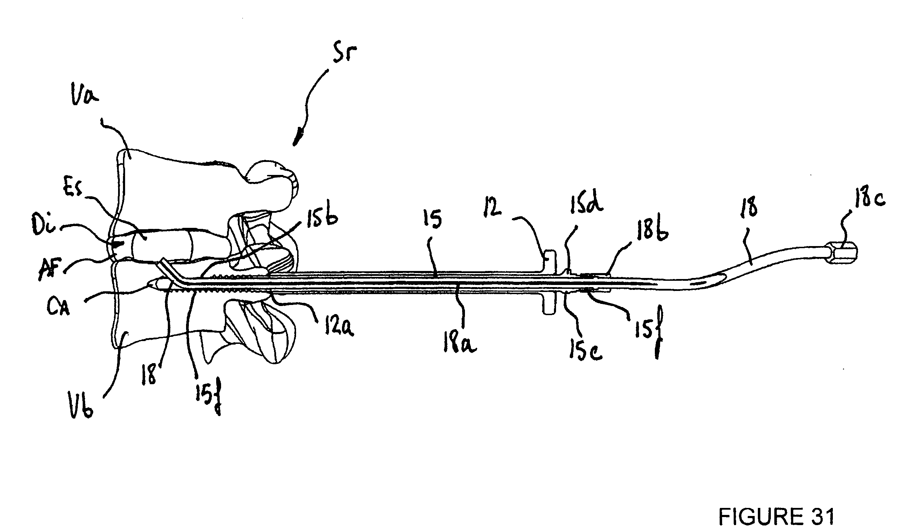

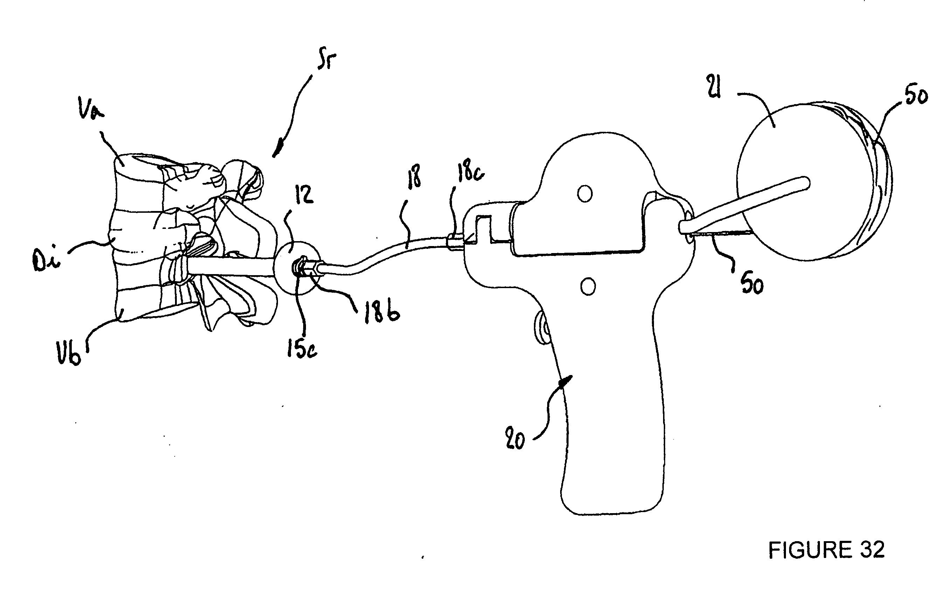

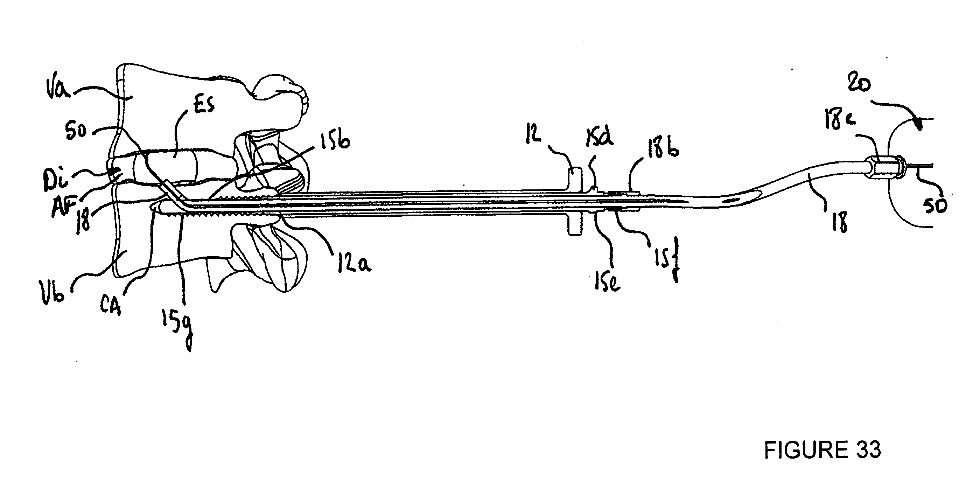

Ainsi, la visée pédiculaire, la nucléotomie du disque intervertébral Di et l'introduction de l'implant nucléique 1 sont conduites de la manière suivante par le chirurgien :
- Le chirurgien positionne et introduit une broche guide 10 sous contrôle radiologique dans le pédicule de la vertèbre sous jacente Vb du segment rachidien Sr (Figure 19).
- Le chirurgien enfile une bougie 11 sur la broche guide 10 pour atteindre le pédicule (Figure 20).
- Le chirurgien positionne sur la bougie 11 un tube guide ou canule droite 12 qui est pourvu à son extrémité de pointes 12a permettant un ancrage dans le corps de la vertèbre sous jacente Vb (Figures 21 à 23).
- Le chirurgien retire la bougie 11 et introduit dans le tube guide ou canule droite 12 un forêt canulé droit 13 permettant de ménager dans le corps de la vertèbre un canal osseux de guidage transpédiculaire Ca jusqu'en dessous du plateau supérieur Ps de la vertèbre sous jacente Vb (Figures 23, 24).
- Le chirurgien retire le foret canulé 13 et la broche 10 pour introduire dans le canal osseux Ca une canule 15 pourvue à l'une de ses extrémités d'un filetage 15g assurant son ancrage osseux. Cette canule filetée 15 présente un canal intérieur 15a se terminant par une sortie latérale 15b destinée à atteindre le plateau supérieur Ps de la vertèbre sous jacente Vb. Le vissage de la canule 15 est réalisé sous contrôle radiologique permettant le repérage du canal 15a qui doit être placé au niveau du centre du disque Di. La canule 15 comporte une extrémité 15c qui dépasse au niveau de la peau de manière que l'entrée du canal 15a soit facilement accessible. L'extrémité de la canule 15 est constituée d'une tête 15c dont le profil externe permet l'entraînement en rotation de ladite canule. La tête 15c est solidaire sur sa périphérie d'un index de repérage 15d permettant au chirurgien de visualiser la position de la sortie latérale 15b du canal intérieur 15a à l'intérieur de la vertèbre sous jacente Vb. La tête 15c comporte un prolongement fileté 15f assurant la connexion de la canule 15 avec une gaine guide fil 18 (Figure 25).
- Le chirurgien positionne l'orifice latéral 15b de la canule filetée15 en direction du plateau supérieur Ps de la vertèbre sous jacente Vb pour pouvoir atteindre la face inférieure du disque intervertébral Di (Figure 26).
- Le chirurgien perfore le plateau supérieur Ps de la vertèbre sous jacente Vb et la face inférieure du disque intervertébral Di au moyen d'un foret ou d'une pointe carrée flexible 16 introduite à l'intérieur de l'alésage 15a de la canule filetée 15 pour atteindre la structure interne désignée « nucleus pulposus » (NP) dudit disque intervertébral Di (Figures 27, 28).
- Le chirurgien procède ensuite à la nucléotomie du disque intervertébral Di. La nucléotomie percutanée consiste à éliminer, de manière contrôlée, le maximum de « nucleus pulposus » (NP) du disque intervertébral Di au travers de la canule filetée 15. Pour cela, le chirurgien insère au moyen de la canule filetée 15 et dans le disque intervertébral Di, un dispositif d'extraction, non représenté, de faible diamètre comprenant, par exemple, une fibre laser, une fibre optique reliée à une caméra, un trou d'irrigation et un trou d'aspiration. Le dispositif d'extraction comporte un mécanisme permettant au chirurgien d'orienter la fibre laser pour réaliser l'élimination du « nucleus pulposus » (NP) de manière précise et sous contrôle de la caméra. Le faisceau laser vaporise les tissus à éliminer et le système d'irrigation intégré dans le dispositif d'extraction permet de maintenir une température contrôlée pour éviter d'affecter les tissus environnant la zone de travail et d'évacuer les produits de la nucléotomie par le trou d'aspiration du dispositif d'extraction. La nucléotomie est réalisée, selon les habitudes de chaque chirurgien, manuellement, au laser ou par coblation. Le chirurgien retire le « nucleus pulposus » (NP) afin de libérer un espace nucléique Es (Figure 29).
- Dès que l'espace nucléique Es est réalisé, vidé et nettoyé, le chirurgien peut procéder à l'introduction de l'implant nucléique 1 constitué de l'élément de remplissage 2 formé du fil 50 à l'intérieur dudit espace.
- Le chirurgien introduit dans la canule filetée 15 une gaine flexible guide fil 18 jusqu'à atteindre le plateau supérieur Ps de la vertèbre sous jacente Vb pour déboucher à l'intérieur de l'espace nucléique Es. La gaine flexible 18 possède un alésage interne 18a qui correspond sensiblement au diamètre de l'élément de remplissage 2 qui peut être constitué d'un fil 50 à introduire dans l'espace nucléique Es. La gaine flexible 18 est fixée à la canule filetée 15 dès que son extrémité libre pénètre légèrement dans l'espace nucléique Es ménagé dans le disque intervertébral Di par l'intermédiaire d'un premier raccord 18b. La gaine flexible 18 a pour fonction de faciliter le guidage du fil 50 jusque dans l'espace nucléique Es libéré par la nucléotomie (Figures 30, 31).
- Le chirurgien connecte la gaine flexible 18 au moyen d'un système d'introduction de type « avance fil dans les postes de soudure » comportant par exemple un pistolet 20 pourvu d'un chargeur interne ou externe 21 dans lequel est enroulée une longueur suffisante de fil 50 (Figure 32).
- Le chirurgien agit sur les moyens d'entraînement du pistolet 20 provoquant une poussée du fil 50 à l'intérieur de l'alésage interne 18a de la première gaine flexible 18 et ce jusqu'à l'intérieur de l'espace nucléique Es ménagé dans le disque intervertébral Di. Une certaine longueur de fil 50 est introduite dans l'espace nucléique Es avec une vitesse contrôlée. L'introduction en continu du fil 50 et son placement en forme de couronne dans l'espace nucléique Es sont suivie sous contrôle radiologique car le fil 50 est radio opaque. (Figure 33).
- Le chirurgien retire la gaine flexible 18, le système d'introduction 20, la canule filetée 15 et le tube guide ou canule droite 12 du canal osseux Ca.
- Le chirurgien fixe l'extrémité du fil 50 constituant la couronne à la vertèbre Vb à l'intérieur du canal osseux Ca par l'intermédiaire de moyens de fixation ou d'un obturateur 43 qui permet également d'obturer ledit canal osseux. Le fil 50 solidarise ainsi l'implant nucléique 1 à la vertèbre Vb pour prévenir les problèmes potentiels de migration.

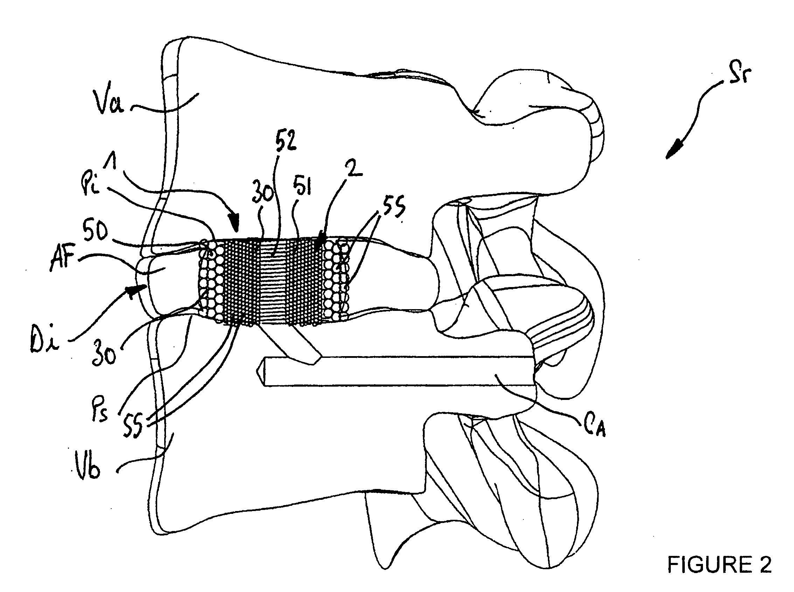

### Implant nucléique 1 à deux fils 50, 51 (figures 2, 34, 36)

Dans le cas ou l'élément de remplissage 2 est formé, par exemple, d'une couronne de fil 50 délimitant un espace interne central 52 ce dernier peut être rempli par un autre fil 51 agencé soit en couronne, soit en pelote. L'élément de remplissage 2 est formé, par exemple, d'une couronne de fil 50 remplissant partiellement l'espace nucléique Es et permettant de réaliser un pseudo annulus, qui aura pour fonction de protéger et renforcer l'« annulus fibrosus » (AF) et éviter les risques de formation ou dégradation ultérieure de « l'annulus fibrosus » (AF).

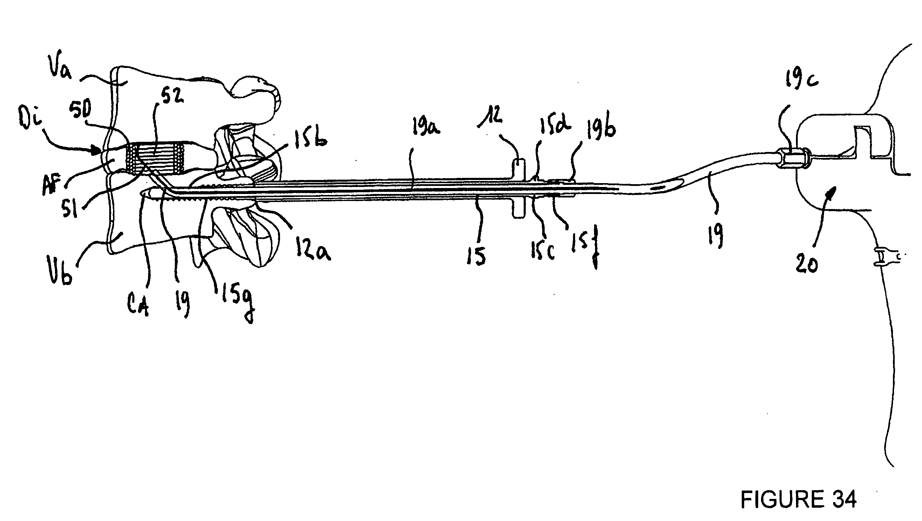

Ainsi, après le retrait de la gaine flexible 18 :
- Le chirurgien introduit une autre gaine flexible guide fil 19 jusqu'à atteindre le plateau de la vertèbre pour déboucher à l'intérieur de l'espace nucléique Es (Figure 34).
- Le chirurgien connecte l'autre gaine flexible guide fil 19 par des raccords 19b, 19c au canon du pistolet 20 pourvu d'un autre chargeur 22 autour duquel est enroulé un second fil 51 dont le diamètre externe est différent de celui du premier fil 50 (Figure 34).
- Le chirurgien agit sur les moyens d'entraînement du pistolet 20 provoquant une poussée du fil 51 à l'intérieur de l'autre gaine flexible 19 et ce jusqu'à l'intérieur de l'espace nucléique Es ménagé dans le disque intervertébral Di. Une certaine longueur de fil 51 est introduite dans l'espace interne central 52 délimité par le fil 50 agencé en couronne. L'introduction en continu du fil 51 et son placement en forme de couronne ou en pelote sont suivie sous contrôle radiologique car le fil 51 est radio opaque (Figures 2, 34, 36).
- Le chirurgien retire la gaine flexible 19, le système d'introduction 20, et les éléments de guidage du canal osseux Ca.
- Le chirurgien fixe l'extrémité des fils 50, 51 à l'intérieur du canal osseux Ca par l'intermédiaire de moyens de fixation ou d'un obturateur 43 qui permet également d'obturer ledit canal osseux. Les fils 50, 51 solidarisent ainsi l'implant nucléique 1 à la vertèbre pour prévenir les problèmes potentiels de migration.

Le second fil 51 est destiné à remplir l'espace nucléique Es restant, c'est à dire, l'espace interne central 52 délimité par la première couronne de fil 50 formant le pseudo annulus. Le second fil 51 est introduit en pression grâce au pistolet 20 pour obtenir un taux de remplissage suffisant nécessaire à une bonne tenue à la compression.

Le second fil 51 est agencé soit suivant une seconde couronne soit suivant une pelote, soit suivant les deux formant un pseudo nucléus. Lorsque la seconde couronne ou la pelote de fil 51 formant le pseudo nucléus est considérée comme satisfaisante à savoir très dense le second fil 51 fixé à l'obturateur 43.

Dans le cas ou l'élément de remplissage 2 est formé, par exemple, d'une couronne de fil 50 délimitant un espace interne central 52 ce dernier peut être rempli soit par un autre fil 51 soit par un produit 53 qui peut être gel ou un produit pâteux ou un produit à base de fibres. L'élément de remplissage 2 est formé, par exemple, d'une couronne de fil 50 et remplissant partiellement l'espace nucléique Es permet de réaliser un pseudo annulus, qui aura pour fonction de protéger et renforcer l'« annulus fibrosus » (AF) et éviter les risques de formation ou dégradation ultérieure de « l'annulus fibrosus » (AF).

### Implant nucléique 1 à fil 50, 51 et un produit 53 de type gel ou pâteux ou à base de fibres ou un matériau viscoélastique injectable.

L'élément de remplissage 2 peut être également réalisé à partir d'un seul fil 50 en forme de couronne dont l'espace interne central 52 et les interstices 30 sont remplis d'un produit 53 qui peut être gel ou un produit pâteux ou un produit à base de fibres ou un matériau viscoélastique injectable formant un « pseudo-nucléus » (Figure 18).

L'implant nucléique 1 ainsi obtenu forme un néo-nucléus ayant de bonnes caractéristiques mécaniques, de cohésion et d'amortissement constitué par la couronne de fil 50 formant un pseudo-annulus et d'un produit 53 qui peut être gel ou un produit pâteux ou un produit à base de fibres ou un matériau viscoélastique injectable formant un « pseudo-nucléus ».

L'élément de remplissage 2 peut être constitué d'une première couronne de fil 50 délimitant un espace central interne 52 et un certain nombre d'interstices 30 entre les spires 55 de l'enroulement, d'un second fil 51 agencé suivant une seconde couronne, et d'un produit 53 qui peut être gel ou un produit pâteux ou un produit à base de fibres ou un matériau viscoélastique injectable formant un « pseudo-nucléus » (Figure 35).

Les interstices 30 et l'espace central interne 52 de l'implant nucléique 1 sont remplis d'un produit 53 qui peut être gel ou un produit pâteux ou un produit à base de fibres ou un matériau viscoélastique injectable.

Il a été observé lorsqu'une compression est exercée sur l'élément de remplissage 2 que ce dernier entraîne des efforts de contact sur les fils 50, 51, les verrouillant entre eux. Ce verrouillage donne à l'élément de remplissage 2 une consistance permettant un bon comportement mécanique aux efforts de compression et une tenue de la structure. Plus cette structure est dense, plus le comportement de l'élément de remplissage 2 est rigide.

Cet élément de remplissage 2 permet d'obtenir une bonne résistance à la compression nécessaire pour un implant nucléique 1 tout en limitant les efforts latéraux. La limitation des efforts latéraux notamment sur « l'annulus fibrosus » (AF) est un point clef pour un tel implant afin d'éviter les phénomènes de compression nerveuse pouvant survenir par le bombement externe de « l'annulus fibrosus » (AF) du disque intervertébral Di ou par une hernie discale.

On note que l'implant nucléique 1 suivant la présente invention peut être associé avec une prothèse facettaire mise en même temps ou en complément lorsque ladite prothèse facettaire est déjà installée sur les vertèbres sus et sous jacentes Va, Vb du segment rachidien Sr.

Les voies d'abord, décrites ci-après, sont données à titre d'exemple non limitatif permettant au chirurgien d'atteindre le « nucleus pulposus » (NP) du disque intervertébral Di.

La voie d'abord transpédiculaire percutanée est réalisée soit par la vertèbre sus jacente Va soit par la vertèbre sous jacente Vb du segment rachidien Sr afin d'atteindre le disque intervertébral Di par forage du pédicule de ladite vertèbre sus jacente Va ou sous jacente Vb.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tous autres équivalents.

## Revendications

1. Implant nucléique constituant, entre deux vertèbres sus et sous jacentes Va, Vb d'un segment rachidien Sr, un dispositif d'étaiement intervertébral amortissant et assurant la mobilité de l'unité fonctionnelle formée par lesdites vertèbres d'une colonne vertébrale, **caractérisé en ce qu'**il est constitué d'au moins un élément de remplissage (2) comportant au moins un fil continu (50, 51) agencé, à l'intérieur d'un espace nucléique Es obtenu après nucléotomie du disque intervertébral Di, suivant un profil en forme de couronne dont l'empilement des spires (55) permet de délimiter un espace interne central (52), tandis que ledit espace interne central (52) est rempli d'un produit (53) qui peut être un gel ou un produit pâteux ou un produit à base de fibres ou un matériau viscoélastique injectable.

2. Implant nucléique suivant la revendication 1, **caractérisé en ce qu'**il est constitué d'un élément de remplissage (2) comportant d'une part un premier fil (50) formant, à l'intérieur d'un espace nucléique Es obtenu après nucléotomie du disque intervertébral Di, une première couronne et d'autre part à l'intérieur de l'espace interne central (52) délimité par la première couronne un second fil (51) agencé pour réaliser une pelote.

3. Implant nucléique suivant la revendication 1, **caractérisé en ce qu'**il est constitué d'un élément de remplissage (2) comportant d'une part un premier fil (50) formant, à l'intérieur d'un espace nucléique Es obtenu après nucléotomie du disque intervertébral Di, une première couronne et d'autre part à l'intérieur de l'espace interne central (52) délimité par la première couronne un second fil (51) agencé pour réaliser une autre couronne.

4. Implant nucléique suivant l'une quelconque des revendications 1 à 3, **caractérisé en ce que** le fil (50, 51) comporte des principes actifs favorisant la formation de fibroses à l'intérieur de l'élément de remplissage (2).

5. Implant nucléique suivant l'une quelconque des revendications 1 à 4, **caractérisé en ce que** le fil (50, 51) est radio opaque permettant de contrôler le remplissage et l'agencement en forme de couronne du fil à l'intérieur de l'espace nucléique Es.

6. Implant nucléique suivant l'une quelconque des revendications 1 à 5, **caractérisé en ce que** le fil (50, 51) est réalisé à partir de matériaux composites ou tressés et bio-résorbables.

7. Implant nucléique suivant l'une quelconque des revendications 1 à 2, **caractérisé en ce que** le fil (50, 51) agencé suivant un profil en forme de couronne présente un axe d'enroulement qui est sensiblement perpendiculaire aux plateaux des vertèbres Va, Vb du segment rachidien Sr.

8. Implant nucléique suivant l'une quelconque des revendications 1 à 7, **caractérisé en ce que** le fil (50, 51) comporte une extrémité libre qui est affinée ou effilée pour éviter les blessures des tissus.

9. Implant nucléique suivant la revendication 1, **caractérisé en ce que** le fil (50, 51) est retenu à la vertèbre Va, Vb par l'intermédiaire d'un obturateur ou tout autre moyen (43) pour éviter la migration dudit fil.

10. Implant nucléique suivant la revendication 1, **caractérisé en ce qu'**il comprend au moins deux couronnes de fil (50) disposées l'une à côte de l'autre dans le même espace nucléique Es.

## Patentansprüche

1. Nucleus-pulposus-Implantat, das zwischen zwei darüber- und darunterliegenden Wirbeln Va, Vb eines Wirbelsäulensegments Sr eine Zwischenwirbelstützvorrichtung darstellt, die Stöße dämpft und die Beweglichkeit der aus den Wirbeln einer Wirbelsäule gebildeten Funktionseinheit gewährleistet, **dadurch gekennzeichnet, dass** es aus mindestens einem Füllelement (2) besteht, das mindestens ein kontinuierliches Filament (50, 51) umfasst, das im Inneren eines nach der Nukleotomie der Bandscheibe Di erhaltenen Nucleuspulposus-Raums Es gemäß einem ringförmigen Profil angeordnet ist, dank dessen Stapel aus Spiralen (55) ein mittlerer Innenraum (52) begrenzt werden kann, während der mittlere Innenraum (52) mit einem Produkt (53) gefüllt ist, bei dem es sich um ein Gel oder ein pastöses Produkt oder um ein Produkt auf Fasergrundlage oder um ein injizierbares viskoelastisches Material handeln kann.

2. Nucleus-pulposus-Implantat nach Anspruch 1, **dadurch gekennzeichnet, dass** es aus einem Füllelement (2) besteht, das einerseits ein erstes Filament (50), das im Inneren eines nach der Nukleotomie der Bandscheibe Di erhaltenen Nucleus-pulposus-Raums Es einen ersten Ring bildet, und andererseits im Inneren des durch den ersten Ring begrenzten mittleren Innenraums (52) ein zweites Filament (51) umfasst, das angeordnet ist, um ein Knäuel zu bilden.

3. Nucleus-pulposus-Implantat nach Anspruch 1, **dadurch gekennzeichnet, dass** es aus einem Füllelement (2) besteht, das einerseits ein erstes Filament (50), das im Inneren eines nach der Nukleotomie der Bandscheibe Di erhaltenen Nucleus-pulposus-Raums Es einen ersten Ring bildet, und andererseits im Inneren des durch den ersten Ring begrenzten mittleren Innenraums (52) ein zweites Filament (51) umfasst, das angeordnet ist, um einen weiteren Ring zu bilden.

4. Nucleus-pulposus-Implantat nach einem der Ansprüche 1 bis 3, **dadurch gekennzeichnet, dass** das Filament (50, 51) Wirkstoffe umfasst, die die Bildung von Fibrosen im Inneren des Füllelements (2) fördern.

5. Nucleus-pulposus-Implantat nach einem der Ansprüche 1 bis 4, **dadurch gekennzeichnet, dass** das Filament (50, 51) röntgendicht ist, wodurch das Füllen und die ringförmige Anordnung des Filaments im Inneren des Nucleus-pulposus-Raums Es gesteuert werden können.

6. Nucleus-pulposus-Implantat nach einem der Ansprüche 1 bis 5, **dadurch gekennzeichnet, dass** das Filament (50, 51) aus Verbundmaterialien oder geflochtenen und bioresorbierbaren Materialien ausgeführt ist.

7. Nucleus-pulposus-Implantat nach einem der Ansprüche 1 bis 2, **dadurch gekennzeichnet, dass** das gemäß einem ringförmigen Profil angeordnete Filament (50, 51) eine Wickelachse aufweist, die im Wesentlichen senkrecht zu den Platten der Wirbel Va, Vb des Wirbelsäulensegments Sr verläuft.

8. Nucleus-pulposus-Implantat nach einem der Ansprüche 1 bis 7, **dadurch gekennzeichnet, dass** das Filament (50, 51) ein freies Ende umfasst, das abgerundet oder verjüngt ist, um Gewebeverletzungen zu vermeiden.

9. Nucleus-pulposus-Implantat nach Anspruch 1, **dadurch gekennzeichnet, dass** das Filament (50, 51) mittels eines Obturators oder eines beliebigen anderen Mittels (43) am Wirbel Va, Vb gehalten ist, um die Migration des Filaments zu verhindern.

10. Nucleus-pulposus-Implantat nach Anspruch 1, **dadurch gekennzeichnet, dass** es mindestens zwei Ringe aus Filament (50) umfasst, die nebeneinander im selben Nucleus-pulposus-Raum Es angeordnet sind.

## Claims

1. Nucleus pulposus implant constituting, arranged between two adjacent vertebrae Va, Vb of a spinal segment Sr, an intervertebral bracing device which absorbs shocks and which ensures mobility of the functional unit formed by said vertebrae of a spinal column, **characterized in that** it consists of at least one filling element (2) comprising at least one continuous filament (50, 51) which is arranged inside a nucleus pulposus space Es obtained after nucleotomy of the intervertebral disk Di, following a ring-shaped profile, of which the stack of spirals (55) makes it possible to delimit a central inner space (52), while said central inner space (52) is filled with a product (53) which may be a gel or a paste product or a product based on fibres or an injectable viscoelastic material.

2. Nucleus pulposus implant according to Claim 1, **characterized in that** it consists of a filling element (2) comprising on the one hand a first filament (50) forming a first ring inside a nucleus pulposus space Es obtained after nucleotomy of the intervertebral disk Di and, on the other hand, a second filament (51) arranged to form a ball inside the central inner space (52) delimited by the first ring.

3. Nucleus pulposus implant according to Claim 1, **characterized in that** it consists of a filling element (2) comprising on the one hand a first filament (50) forming a first ring inside a nucleus pulposus space Es obtained after nucleotomy of the intervertebral disk Di and, on the other hand, a second filament (51) arranged to form another ring inside the central inner space (52) delimited by the first ring.

4. Nucleus pulposus implant according to any one of Claims 1 to 3, **characterized in that** the filament (50, 51) comprises active principles promoting the formation of fibroses inside the filling element (2).

5. Nucleus pulposus implant according to any one of Claims 1 to 4, **characterized in that** the filament (50, 51) is radiopaque, making it possible to monitor the filling and the ring-shaped arrangement of the filament inside the nucleus pulposus space Es.

6. Nucleus pulposus implant according to any one of Claims 1 to 5, **characterized in that** the filament (50, 51) is made from composite or braided and bioabsorbable materials.

7. Nucleus pulposus implant according to either of Claims 1 and 2, **characterized in that** the filament (50, 51) arranged following a ring-shaped profile has a winding axis which is substantially perpendicular to the plates of the vertebrae Va, Vb of the spinal segment Sr.

8. Nucleus pulposus implant according to any one of Claims 1 to 7, **characterized in that** the filament (50, 51) comprises a free end which is narrowed or tapered in order to avoid injury to the tissues.

9. Nucleus pulposus implant according to Claim 1, **characterized in that** the filament (50, 51) is retained on the vertebrae Va, Vb using an obturator or any other means (43) for preventing migration of said filament.

10. Nucleus pulposus implant according to Claim 1, **characterized in that** it comprises at least two filament rings (50) arranged beside one another in the same nucleus pulposus space Es.
